# EUROPEAN PATENT APPLICATION

(11) **EP 4 272 681 A1**
(43) Date of publication of application: **08.11.2023**
(21) Application number: 23170832.2
(22) Date of filing: 28.04.2023
(51) Int. Cl.: A61B 34/30, G16H 40/63, A61B 90/50, A61B 17/00

(54) **SYSTEM AND METHOD FOR RADIO-BASED LOCALIZATION OF COMPONENTS IN A SURGICAL ROBOTIC SYSTEM**

(30) Priority: 03.05.2022 US 202263337633 P; 10.04.2023 US 202318132552
(71) Applicant: Covidien LP, Mansfield, MA 02048 (US)
(72) Inventor: PEINE, William J., Boston, 02210 (US)
(74) Representative: Maschio, Antonio

(57) **Abstract**

A position and tracking system for radio-based localization and data exchange includes a radio receiver, a mobile cart, a processor, and a memory coupled to the processor. The mobile cart includes a robotic arm and a radio transmitter in operable communication with the radio receiver. The memory has instructions stored thereon which, when executed by the processor, cause the system to receive, from the radio transmitter, a signal including communication data and localization data of the mobile cart in a 3D space, determine a destination for the mobile cart based on the communication data in the received signal, and cause the mobile cart to move to the destination determined.

## Description

### CROSS-REFERENCE TO RELATED APPLICATIONS

The present application claims the benefit of and priority to U.S. Provisional Patent Application Serial No. 63/337,633, filed on May 3, 2022, the entire contents of which being incorporated herein by reference.

### FIELD

The present disclosure generally relates to a surgical robotic system having one or more modular arm carts each of which supports a robotic arm. More particularly, the present disclosure is directed to a system and method for radio-based localization and data exchange of the mobile modular arm carts in a surgical robotic system in three-dimensional space.

### BACKGROUND

Surgical robotic systems have become widely used by surgeons in surgical procedures because these systems enable surgery to be less invasive as compared to conventional open surgical procedures in which the surgeon is required to cut open large areas of body tissue. As a direct result thereof, robotic surgical systems minimize trauma to the patient and reduce patient recovery time and hospital costs. A hospital or surgical center may operate a surgical robotic system with multiple robotic arms. Knowing where the robotic arms are located and tracking their usage may be difficult.

### SUMMARY

In accordance with aspects of the disclosure, a position and tracking system for radio-based localization and data exchange includes a radio receiver, a mobile cart, a processor, and a memory coupled to the processor. The mobile cart includes a robotic arm and a radio transmitter in operable communication with the radio receiver. The memory has instructions stored thereon which, when executed by the processor, cause the system to receive, from the radio transmitter, a signal including communication data and localization data of the mobile cart in a 3D space, determine a destination for the mobile cart based on the communication data in the received signal, and cause the mobile cart to move to the destination determined.

In an aspect, the communication data includes at least one of a specific surgical procedure, a specific type of patient, a specific type of surgical table, or the configuration of an operating room. Additionally or alternatively, the instructions, when executed, further cause the system to determine the destination for the mobile cart based on at least one of a specific surgical procedure, a specific type of patient, a specific type of surgical table, or the configuration of an operating room, and cause the mobile cart to move to a new spatial pose.

In an aspect, the signal includes communication data in parallel to the localization data.

In an aspect, the mobile cart includes a battery power supply. Additionally, or alternatively, the communication data includes data corresponding to a power level of the battery power supply. Additionally, or alternatively, the instructions, when executed, further cause the system to determine whether the power level of the battery power supply is below a preconfigured threshold, and cause the mobile cart to move to a charging station when it is determined that the power level of the battery power supply is below the preconfigured threshold.

In an aspect, the communication data includes data corresponding to service data of the mobile cart. Additionally, or alternatively, the instructions, when executed, further cause the system to determine whether the mobile cart requires servicing based on the communication data, and cause the mobile cart to move to a servicing center when it is determined that the mobile cart requires servicing.

In an aspect, the instructions, when executed, further cause the system to register the mobile cart to other mobile carts and to a surgical table based on the communication data and localization data of the mobile cart.

In an aspect, the instructions, when executed, further cause the system to register the mobile cart to pre-operative data based on the communication data and localization data of the mobile cart.

In an aspect, the instructions, when executed, further cause the system to adjust data storage parameters or data communication parameters based on the communication data and localization data of the mobile cart.

According to another aspect of the disclosure, a method for radio-based localization and data exchange is provided. The method includes receiving, from a radio transmitter, a signal including communication data and localization data of a mobile cart in a 3D space, wherein the communication data includes data corresponding to a power supply level of the mobile cart and data corresponding to service data of the mobile cart, determining a destination for the mobile cart based on the communication data in the received signal, causing the mobile cart to move to a charging station when it is determined that a power supply of the mobile cart is below a threshold, and causing the mobile cart to move to a servicing center when it is determined that the mobile cart requires servicing.

In an aspect, the communication data includes data corresponding to a specific surgical procedure, a specific type of patient, a specific type of surgical table, or the configuration of an operating room, and determining the destination for the mobile cart includes determination the destination based on the data corresponding to a specific surgical procedure, a specific type of patient, a specific type of surgical table, or the configuration of an operating room.

In an aspect, the method further includes registering the mobile cart to other mobile carts and to a surgical table based on the communication data and localization data of the mobile cart.

In an aspect, the method further includes registering the mobile cart to pre-operative data based on the communication data and localization data of the mobile cart.

In an aspect, the method further includes adjusting data storage parameters or data communication parameters based on the communication data and the localization data of the mobile cart.

According to another aspect of the disclosure, a non-transitory computer-readable storage medium is provided. The non-transitory computer-readable storage medium stores a program, which when executed by a computer, causes the computer to receive, from a radio transmitter, a signal including communication data and localization data of a mobile cart in a 3D space, wherein the communication data includes data corresponding to a power supply level of the mobile cart, determine a destination for the mobile cart based on the communication data in the received signal, cause the mobile cart to move to a charging station when it is determined that a power supply of the mobile cart is below a threshold, and adjust data storage parameters or data communication parameters based on the communication data and the localization data of the mobile cart.

In an aspect, the communication data includes at least one of a specific surgical procedure, a specific type of patient, a specific type of surgical table, or the configuration of an operating room and the program, when executed by the computer, causes the computer to determine the destination based on at least one of a specific surgical procedure, a specific type of patient, a specific type of surgical table, or the configuration of an operating room.

In an aspect, the program, when executed by the computer, causes the computer to register the mobile cart to other mobile carts and to a surgical table based on the communication data and localization data of the mobile cart.

In an aspect, the program, when executed by the computer, causes the computer to register the mobile cart to pre-operative data based on the communication data and localization data of the mobile cart.

In an aspect, the communication data includes data corresponding to service data of the mobile cart and the program, when executed by the computer, causes the computer to determination the destination based on the data corresponding to service data of the mobile cart.

In an aspect, the program, when executed by the computer, causes the computer to localize and register a position of a surgical instrument to intraoperative imaging based on the communication data and localization data of the mobile cart.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

Various aspects of the present disclosure are described herein with reference to the drawings wherein:
FIG. 1 is a schematic illustration of a surgical robotic system including a control tower, a console, and one or more surgical robotic arms according to the present disclosure;
FIG. 2 is a perspective view of a surgical robotic arm of the surgical robotic system of FIG. 1 according to the present disclosure;
FIG. 3 is a perspective view of a setup arm with the surgical robotic arm of the surgical robotic system of FIG. 1 according to the present disclosure;
FIG. 4 is a schematic diagram of a computer architecture of the surgical robotic system of FIG. 1 according to the present disclosure; and
FIG. 5 is a flowchart of a method for radio-based data exchange and localization of components of a surgical robotic system in accordance with the disclosure.

### DETAILED DESCRIPTION

Embodiments of the presently disclosed surgical robotic systems are described in detail with reference to the drawings, in which like reference numerals designate identical or corresponding elements in each of the several views. As used herein, the term "distal" refers to the portion of the surgical robotic system and/or the surgical instrument coupled thereto that is closer to the patient, while the term "proximal" refers to the portion that is farther from the patient.

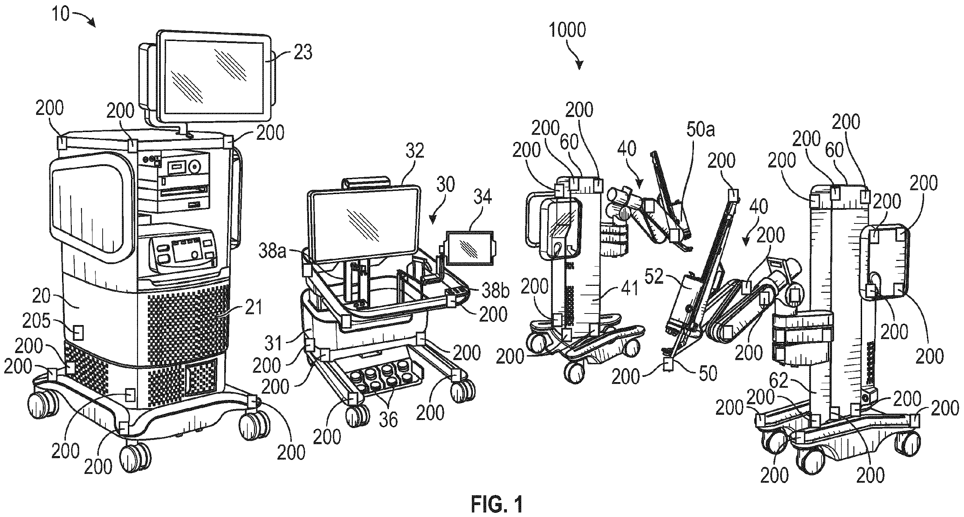

Although the following description is specific to a surgical robotic system, the radio-based location system described below may be used with any suitable medical device, such as hand-held surgical instruments or components thereof, requiring an alignment relative to a representative coordinate system or another orientation point, or tracking throughout a facility. With reference to FIG. 1, a surgical robotic system 10 includes a control tower 20, which is connected to all of the components of the surgical robotic system 10, including a surgical console 30 and one or more robotic arms 40. Each of the robotic arms 40 includes a surgical instrument 50 removably coupled thereto. One or more of the robotic arms 40 may include an endoscope or a camera for observing the surgical site. The surgical instrument 50 is configured for use during minimally invasive surgical procedures. In embodiments, the surgical instrument 50 may be configured for open surgical procedures. Each of the robotic arms 40 is also coupled to a mobile cart 60.

The surgical console 30 includes a first display device 32, which displays a surgical site provided by cameras (not shown) disposed on the robotic arms 40, and a second display device 34, which displays a user interface for controlling the surgical robotic system 10. The surgical console 30 also includes a plurality of user interface devices, such as foot pedals 36 and a pair of handle controllers 38a and 38b, which are used by a clinician to remotely control robotic arms 40.

The control tower 20 acts as an interface between the surgical console 30 and one or more robotic arms 40. In particular, the control tower 20 is configured to control the robotic arms 40, such as to move the robotic arms 40 and the corresponding surgical instruments 50, based on a set of programmable instructions and/or input commands from the surgical console 30, in such a way that robotic arms 40 and the surgical instrument 50 execute a desired movement sequence in response to input from the foot pedals 36 and the handle controllers 38a and 38b. The control tower 20 includes a display 23 for displaying various information pertaining to the surgical robotic system 10.

Each of the control tower 20, the surgical console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area networks, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 802.15.4-2003 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein. Each of the control tower 20, the surgical console 30, and the robotic arm 40 includes a respective radio transmitter 200. It is contemplated that multiple radio transmitters 200 may be used.

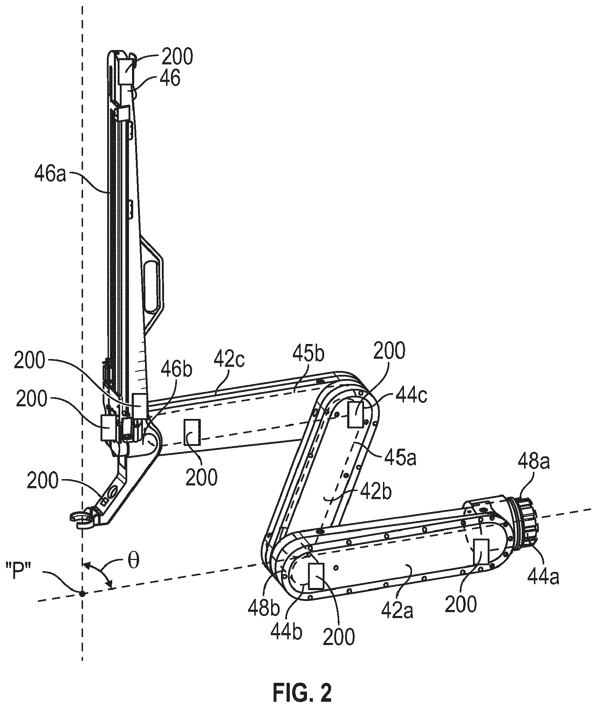

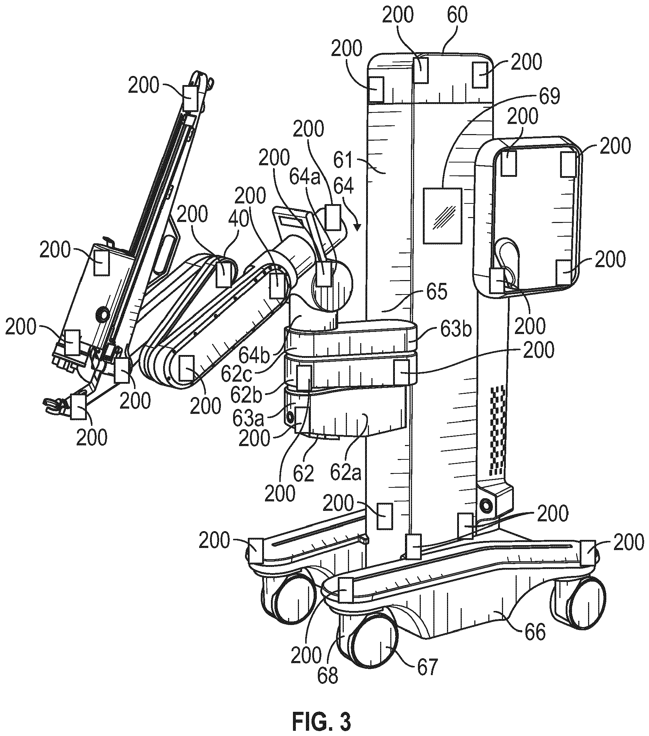

With reference to FIG. 2, each of the robotic arms 40 may include of a plurality of links 42a, 42b, 42c, which are interconnected at rotational joints 44a, 44b, 44c, respectively. The joint 44a is configured to secure the robotic arm 40 to the mobile cart 60 and defines a first longitudinal axis. With reference to FIG. 3, the mobile cart 60 includes a lift 61 and a setup arm 62, which provides a base for mounting of the robotic arm 40. The lift 61 allows for vertical movement of the setup arm 62. The mobile cart 60 includes a base 66 having a plurality of wheels 67, each of which having a brake 68. The mobile cart 60 also includes the cart display 69 for displaying information pertaining to the robotic arm 40.

The setup arm 62 includes a first link 62a, a second link 62b, and a third link 62c, which provide for lateral maneuverability of the robotic arm 40. The links 62a, 62b, 62c are interconnected at rotational joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In embodiments, the robotic arm 40 may be coupled to the surgical table (not shown). The setup arm 62 includes controls 65 for adjusting movement of the links 62a, 62b, 62c as well as the lift 61.

The third link 62c includes a rotatable base 64 having two degrees of freedom. In particular, the rotatable base 64 includes a first actuator 64a and a second actuator 64b. The first actuator 64a is rotatable about a first stationary arm axis, which is perpendicular to a plane defined by the third link 62c, and the second actuator 64b is rotatable about a second stationary arm axis which is transverse to the first stationary arm axis. The first and second actuators 64a and 64b allow for full three-dimensional orientation of the robotic arm 40.

With reference to FIG. 2, the robotic arm 40 also includes a holder 46 defining a second longitudinal axis and configured to receive an instrument drive unit 52 (FIG. 1) of the surgical instrument 50, which is configured to couple to an actuation mechanism of the surgical instrument 50. Instrument drive unit 52 transfers actuation forces from its actuators to the surgical instrument 50 to actuate components (e.g., end effectors) of the surgical instrument 50. The holder 46 includes a sliding mechanism 46a, which is configured to move the instrument drive unit 52 along the second longitudinal axis defined by the holder 46. The holder 46 also includes a rotational joint 46b, which rotates the holder 46 relative to the link 42c.

The joints 44a and 44b include an electrical actuator 48a and 48b configured to drive the joints 44a, 44b, 44c relative to each other through a series of belts 45a and 45b or other mechanical linkages such as a drive rod, a cable, or a lever and the like. In particular, the actuator 48b of the joint 44b is coupled to the joint 44c via the belt 45a, and the joint 44c is in turn, coupled to the joint 46c via the belt 45b. Joint 44c may include a transfer case coupling the belts 45a and 45b, such that the actuator 48b is configured to rotate each of the links 42b, 42c and the holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. Thus, the actuator 48b controls the pitch angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle θ. In embodiments, some or all of the joints 44a, 44b, 44c may include an electrical actuator to obviate the need for mechanical linkages.

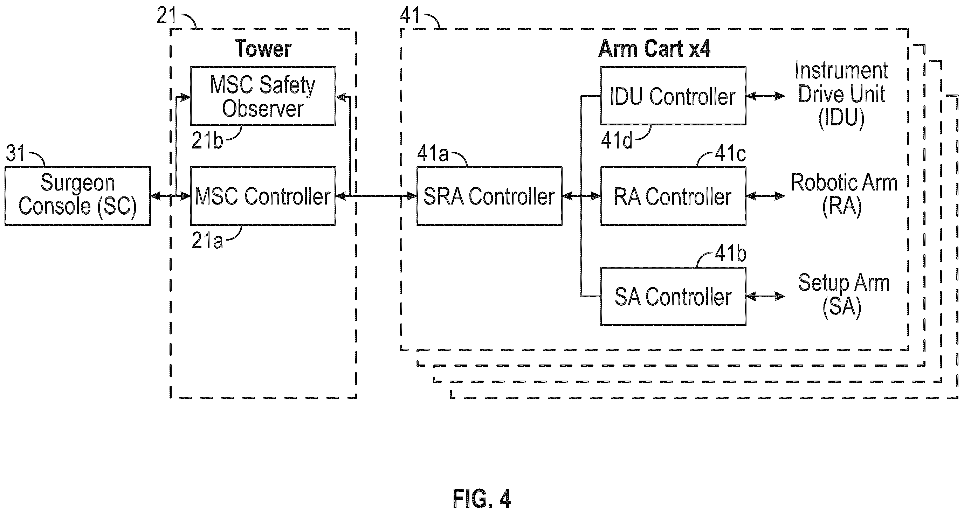

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgical console 30 about the current position and/or orientation of the handle controllers 38a and 38b and the state of the foot pedals 36 and other buttons. The controller 21a processes these input positions to determine desired drive commands for each joint of the robotic arm 40 and/or the instrument drive unit 52 and communicates these to the computer 41 of the robotic arm 40. The controller 21a also receives back the actual joint angles and uses this information to determine force feedback commands that are transmitted back to the computer 31 of the surgical console 30 to provide haptic feedback through the handle controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the mobile cart 60, the robotic arm 40, and the instrument drive unit 52. The main cart controller 41a also communicates actual joint angles back to the controller 21a.

The setup arm controller 41b controls each of rotational joints 63a and 63b, and the rotatable base 64 of the setup arm 62 and calculates desired motor movement commands (e.g., motor torque) for the pitch axis and controls the brakes. The robotic arm controller 41c controls each joint 44a and 44b of the robotic arm 40 and calculates desired motor torques required for gravity compensation, friction compensation, and closed-loop position control. The robotic arm controller 41c calculates a movement command based on the calculated torque. The calculated motor commands are then communicated to one or more of the electrical actuators 48a and 48b in the robotic arm 40. The actual joint positions are then transmitted by the electrical actuators 48a and 48b back to the robotic arm controller 41c.

The IDU controller 41d receives desired joint angles for the surgical instrument 50, such as wrist and jaw angles, and computes desired currents for the motors in the instrument drive unit 52. The IDU controller 41d calculates actual angles based on the motor positions and transmits the actual angles back to the main controller 41a.

The robotic arm controller 41c is configured to estimate torque imparted on the rotational joints 44a and 44b by the rigid link structure of the robotic arm 40, namely, the links 42a, 42b, 42c. Each of the rotational joints 44a and 44b houses electrical actuator 48a and 48b. High torque may be used to move the robotic arm 40 due to the heavy weight of the robotic arm 40. However, the torque may need to be adjusted to prevent damage or injury. This is particularly useful for limiting torque during collisions of the robotic arm 40 with external objects, such as other robotic arms, patient, staff, operating room (OR) equipment, *etc.*

There are many situations in surgical robotics where knowing the position and/or orientation of one or more objects relative to another, or the location of surgical components within a hospital setting, provides insight to enhance clinical performance. Next generation wireless communication technologies, such as 5G and 6G, use high frequency, small wavelength RF signals. This allows for localization of the wireless transmitters in parallel to the transmission of communication data. These wireless networks also allow for small cell sizes, even down to a single hospital, wing, or room. There are many applications of localization technology needed for surgical devices and surgical robotics. These applications include meter-level localization, such as tracking robotics components within the hospital. Other applications require centimeter-level localization (e.g., within a particular space such as an operating room) such as understanding the relative position of the robotic arms 40 or mobile carts 60 relative to each other and the patient. Finally, some applications require millimeter-level localization accuracy, such as tracking tip instruments for automation or registration with pre-operative plans.

Utilizing the localization capabilities of the wireless communications is advantageous because it reduces the complexity of the system since the communication channels can also be used to transmit commands and measured signals to and from other parts of the system. Using this dual functionality obviates the need for other localization methods for redundancy checks, such as optical or magnet tracking.

Another advantage of these new wireless communication systems is the small size of receiver and transmitter hardware, since they have been optimized for small-size and lower-power applications. This small size and reduced power consumption allow the communication hardware to be placed much closer to, or embedded with, the distal sensor or actuator of the surgical instruments 50. This close proximity also reduces wiring complexity and allows for the precise location tracking of multiple parts of the system 10.

In accordance with the present disclosure, a position and tracking system 1000 for an absolute spatial position and pose tracking in surgical robotics including radio frequency sources and receivers includes a radio transmitter 200 and a radio receiver 205. The tracking system 100 is configured for use with, or incorporated into, a robotic surgical system 10, as shown in FIG 1. The radio receiver 205 may include an RF receiver, a microwave receiver, and/or a millimeter-wave receiver. The radio receiver 205 may communicate with the computers 21, 31, 41 of FIG. 1. Briefly, the radio receiver 205 may include a processor (not shown) and memory (not shown). The radio receiver 205 may be located on control tower 20 of FIG. 1. It is contemplated that the radio receiver 205 may be integrated into the ceiling or integrated into the operating room.

The tracking system 1000 may utilize meter-level localization, for example, when tracking components of the robotic system 10 within the hospital, centimeter-level localization, for example, when tracking components within the operating room (e.g., to determine the relative positions of the robotic arms to each other and to the patient), and millimeter-level localization, for example, for tracking the position of the tips of surgical instruments 50 for automation or registration with pre-operative plans.

The radio transmitter 200 may also include an RF transmitter, a microwave transmitter, and/or a millimeter-wave transmitter. In various aspects, a location of radio transmitter 200 (*e.g.*, tracking units, beacons, or sensors) is desired to be tracked with millimeter precision relative to a radio transmitter 200. In various aspects, the position and tracking system 1000 may include one or more radio transmitters 200. In various aspects, the position and tracking system 1000 may include three or more of these radio transmitters 200, allowing the position and tracking system 1000 to determine and monitor the spatial pose of the component to which they are mounted (e.g., surgical instrument 50, mobile cart 60, robotic arm 40, etc.). In embodiments, the position of the control tower 20 of FIG. 1 may be monitored in relation to a patient or the robotic arm 40, and vice-versa. In various aspects, the positions and poses of objects may be remotely monitored by the radio receiver 205 of the position and tracking system 1000, enabling the exemplary use cases described below. In various aspects, the position and tracking system 1000 determines an item's location in 3D based on data communicated by the radio transmitter 200.

In aspects, the radio transmitter 200 is a transceiver and may operate on a 5G network. 5G can be implemented in low-band, mid-band or high-band millimeter-wave 24 GHz up to 54 GHz.

In various aspects, the radio transmitters 200 may be mounted on and throughout the surgical robotic system 10, e.g., the spatial location of subcomponents of the surgical robotic system 10 can be monitored at all times including the mobile cart 60 of the robotic arms 40 as well as the individual links 42a, 42b, 42c of the arms even when they are within their sterile drapes. (*See, e.g.,* FIG. 2 and FIG. 3.) With this capability, placement of the mobile cart 60, to optimal locations, can be ensured with the use of active guidance feedback, for a specific surgical procedure, for a specific type of patient, on the specific type of surgical table, in a specific configuration of an OR.

In accordance with this disclosure, the position and tracking system 1000 may be used to track the positions of the end effectors (or the tip) of the surgical instruments 50 for purposes of better accuracy. Currently, the instrument tip position is estimated based on the joint angles of the robotic arm 40. Additionally, bending of the shaft of the surgical instrument 50 or inaccurate joint angles add up and degrade accuracy. Accordingly, tracking the tip of the surgical instrument 50 provide a level of accuracy that is below millimeter dimensions and allows for image-guided procedures, surgical automation, and force estimates by estimating the bending of the surgical instrument 50.

In various aspects, by placing radio transmitters 200 at various locations on the robotic arm 40 and/or the mobile cart 60, and specifically along the individual links 42a, 42b, 42c of robotic arm 40, the individual links 42a, 42b, 42c of the robotic arm 40 may be constantly monitored, allowing the position and tracking system 1000 to know the locations, orientations and poses of all robotic arms 40 relative to one another at all times. In various aspects, this may be used as a verification to ensure nothing has modified the state of the geometry of radio transmitters 200 on the robotic arm 40. Knowing where all the components of the robotic arm 40 are located relative to one another and adding the shape of those components to the known pose information, potential collisions of the robotic arms 40 can be detected and movements of the robotic arms 40 can be modified, the surgeon can be alerted that corrective action should be taken prior to a collision, or the robotic arm 40 movements can be halted to prevent the collision. In various aspects, the position and tracking system 1000 may use the known spatial pose information to determine the patient, operating table, and/or surgical personnel poses. In various aspects, the position and tracking system 1000 may provide collision avoidance based on at least one radio transmitter 200 on each the robotic arm 40 and at least one radio transmitter 200 on a surgical assistant or the patient.

In various aspects, radio transmitters 200 may be placed on or incorporated into surgical instrument ports 50a (FIG. 2). Specifically, in various aspects, the position and tracking system 1000 may include a radio transmitter 200 integrated into the access port or trocar (not shown) that is inserted into a patient's abdominal cavity. In various aspects, the position and tracking system 1000 may determine location information of the trocar or the access port to use as a setup guide. The locations of surgical instrument ports may be used by the position and tracking system 1000 to perform initial docking of the robotic system 10 to the surgical instrument ports. This can be performed under interactive guidance, which may be used by the OR team becomes familiar with the robotic surgical system 10. In addition, interactive guidance to, and confirmation of optimal placement for surgical ports for a specific patient and procedure will be possible. The position and tracking system 1000 is also configured to continuously monitor and assess surgical instrument port movement, due to the deformation of patient tissue surrounding the surgical instrument port, and guidance provided by the robotic system 10 should excessive tissue movement be found.

In various aspects, the position and tracking system 1000 may track surgical tools (e.g., surgical instruments 50) used during surgery based on radio transmitters 200 located on/in the surgical tools. In various aspects, a radio transmitter 200 may be located at a surgical tool tip of a surgical instrument 50 or on a known location of the surgical instrument 50, and by using the distance and relative positioning therebetween, a location of the surgical tool tip can be determined. For example, with the pose of a surgical instrument port and a pose of the last link 42c of the robotic arm 42 known and combined with the known kinematic state of the surgical tool, endpoint feedback of the surgical tool tips can be determined and continuously monitored such that the relative pose of all the surgical instruments relative to one another as well as relative to an endoscope, can be monitored. This provides an accurate and direct means of tool-tool and tool-camera pose monitoring.

In various aspects, surgical personnel may wear a single radio transmitter 200. In various aspects, the position and tracking system 1000 may determine and monitor personnel actions throughout a procedure to allow datasets to be built to provide predictive monitoring of surgery progress as well as detection of deviation from normative progress with appropriate notification of resources to ensure appropriate actions are taken. In various aspects, personnel may wear multiple radio transmitters 200. In various aspects, the position and tracking system 1000 may determine fine-grained detection of a possible collision with a robotic arm 40 and suggest remedial actions.

In various aspects, the wearing of multiple radio transmitters 200 also allows movements/gestures of personnel to be used as input to control communication amongst personnel using interactive methods such as augmented reality. In various aspects, radio transmitters 200 may be placed on the hands and/or feet of the surgeon, and the position and tracking system 1000 may monitor the movements of hands and/or feet of the surgeon, as a form of input to control/command the surgical robotic system 10, as an alternative to, or as an enhancement of linkage-based command input. In various aspects, the position and tracking system 1000 may include multiple radio transmitters 200 worn by multiple personnel involved in providing control inputs in complex operations. For example, the surgeon may wear a radio transmitter 200 on their foot to use as a virtual foot pedal. In various aspects, the position and tracking system 1000 may include one or more radio transmitters 200 on the surgeon to determine a location and/or orientation of the surgeon. In various aspects, the position and tracking system 1000 may use the location information to ensure that the surgeon is in the field of view of the user interface monitor and facing the screen. In various aspects, a radio transmitter 200 may be integrated on the glasses worn by the surgeon.

In various aspects, radio transmitters 200 may be placed on specific locations on a patient on the operating table. In this manner, patient location on the operating table can be known and confirmed, and this information can be combined with the surgical port tracking, robotic component tracking, and surgical personnel tracking. Benefits of such a complete and continuous spatial information portrait of the operating area include safety monitoring of the movements of the robotic arm 40 movements in relation to all aspects of personnel and equipment in the vicinity thereof. This information also allows adaption of movements of the robotic arms 40 to allow the operating table to be adjusted in the midst of a surgical procedure, thus saving time and enabling new types of surgical site access.

In various aspects, the position and tracking system 1000 may provide setup guidance for robotic system 10. For example, a radio transmitter 200 may be located on the patient, an operating table and/or one or more on each robotic arm 40 and mobile cart 60. In various aspects, one or more radio transmitters 200 may be located on various sections of the operating table. For example, the position and tracking system 1000 can utilize the location information based on the data communicated from the radio transmitter 200 for operating table orientation.

In various aspects, the position and tracking system 1000 may locate robotic arms 40 and/or mobile carts 60 around the hospital. The position and tracking system 1000 may determine that a robotic arm 40 or mobile cart 60 needed in the OR is currently located in a storage closet. Because the position and tracking system 1000 is capable of localization and command data transmission, in aspects, the position and tracking system 1000 may track usage data of the components of the robotic surgical system 10, such as the surgical instrument 50, robotic arm 40, and mobile cart 60. The position and tracking system 1000 may additionally, or alternatively, cause or allow a battery powered mobile cart 60 to drive itself to a charging station when the battery level falls below a preconfigured threshold and/or when other conditions are met.

In various aspects, the position and tracking system 1000 may include radio transmitters 200 integrated into a wand that the surgeon can use to register parts or locations of a patient's anatomy. This can then be used for virtual walls and also aligning pre-operative scans to the user interface 700 and/or operating room team interface (ORTI) endoscope feeds. For example, the surgeon may touch the wand to an anatomical feature of a patient and press a button on the wand indicating the location of the feature.

In various aspects, the radio receiver may include a plurality of antennae. In various aspects, the radio receiver 205 transmits an RF signal (or a millimeter signal or a microwave signal) that is received by the radio receiver. In various aspects, the signal may be a spread spectrum signal. Spread spectrum is a form of wireless communications in which the frequency of the transmitted signal is deliberately varied. For example, a radio transmitter 200 may be located on a robotic arm 40 and may transmit a beacon at 30GHz. It is contemplated that other frequencies may be used. The radio receiver 205, which may be located on the control tower 20, would receive the 30GHz beacon signal from one or more radio transmitters 200 via the plurality of antennae of the radio receiver 205. The position and tracking system 1000 may utilize the level of the 30GHz signal received at each of its antennae to determine the position data for the radio transmitter 200 in the OR. For example, the position and tracking system 1000 may determine the location data for the radio transmitter 200 based on triangulation. The position and tracking system 1000 may take this position data and cross-reference it with kinematic information and/or camera positioning information.

It is additionally contemplated, and within the scope of this disclosure, that if the radio transmitters 200 are sufficiently small, and if the radio transmitters 200 are wireless (or tethered by only a thin wire), then the radio transmitters 200 may be placed directly within the patient's anatomy for tracking the position of organs and other anatomical structures. This could be used for image-guided surgery, updating deformable tissue models, and surgical automation since movement of the tissue, organ or anatomical structure of the patient is known.

While radio receiver 205 of the position and tracking system 1000 is described as being located on control tower 20, it is contemplated that radio receiver 205 of the position and tracking system 1000 may be located in the surgical console 30, in the operating table, or anywhere in/on the OR arena including the ceiling or walls.

It will be understood that various modifications may be made to the embodiments disclosed herein. In embodiments, the radio transmitters 200 of the position and tracking system 1000 may be disposed on any suitable portion of the robotic arm 40. Therefore, the above description should not be construed as limiting, but merely as exemplifications of various aspects. Those skilled in the art will envision other modifications within the scope and spirit of the claims appended thereto.

In various aspects, the position and tracking system 1000 may be used as a capital management tool for hospitals to improve efficiency and/or increase utilization of equipment. For example, the position and tracking system 1000 may track the position and/or usage of all the system components across a hospital. The position and tracking system 1000 may record information regarding usage, downtime, location, and/or managers to help with utilization the modularity of a system. The recorded information may be input into a machine learning module as inputs to predict setup optimization. The position and tracking system 1000 may generate a report regarding the recorded information. For example, the position and tracking system 1000 may have a plurality of receivers located across the hospital, configured to locate a robotic arm 40 anywhere in a hospital. This may reduce downtime between surgeries by allowing hospital staff to locate the nearest unused robotic arm 40. The position and tracking system 1000 may receive a request for an unused robotic arm 40 and provide a message to a user device, or a computing system indicating the location of the robotic arm 40 in the hospital. In various aspects, the robotic arm 40 may further include a GPS receiver and transmit the GPS data to the receiver. In aspects, the robotic arm 40 may include a beacon or a "find me" module. The robotic arm 40 may determine when a battery is below a threshold value and transmit the beacon and/or a message to a user based on the low battery. In various aspects, the position and tracking system 1000 may generate a spaghetti plot to visualize how equipment moves over a time period. The data may be analyzed by machine learning to provide a suggestion for more efficient use of the equipment (e.g., a robotic arm). In various aspects, the position and tracking system 1000 may compare surgical teams to determine best practices and share insights on more efficient use of the equipment. It is contemplated that the disclosed technology could be used to track devices other than medical devices, e.g., a copier for uses such as capital equipment tracking.

Operating room time is valuable. In aspects of the disclosure, the position and tracking system 1000 may be used to simplify the set-up process, cut down on OR turnover time, and reduce trip hazards. For example, the surgical table may include a radio transmitter 200. The type of procedure, habitus, and/or surgery for a patient may be used by the system 1000 to determine where ports need to go as well as where the robotic arm 40 should be located in the OR. For example, the data from the location and timing of the robotic arms 40 and mobile carts 60 may be recorded over time, over multiple procedures. This data may be used as training data for a machine learning network. In aspects, machine learning may be used for the determining. In an aspect, each of the robotic arms 40 may be assigned unique identification numbers based on their location around the patient. In an aspect, the system 1000 may automatically send the mobile carts 60 to an OR based on a scheduled procedure based on the predictions from the machine learning network. The machine learning network may include a neural network and/or a classifier. The machine learning network may predict, based on the training data and the type of procedure which OR will require which robotic arm 40. The mobile carts 60 and robotic arms 40 may automatically locate themselves around the surgical table in the proper OR based on the machine learning network.

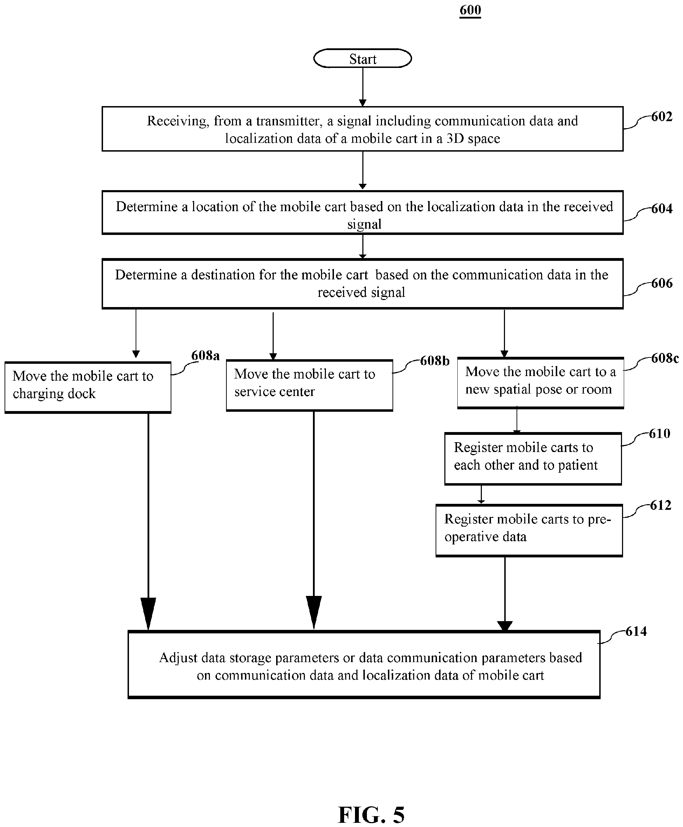

The flow diagram of FIG. 5 described below includes various blocks described in an ordered sequence. However, those skilled in the art will appreciate that one or more blocks of the flow diagram may be performed in a different order, repeated, and/or omitted without departing from the scope of the disclosure. The below description of the flow diagram refers to various actions or tasks performed by the position and tracking system 1000, but those skilled in the art will appreciate that the position and tracking system 1000 is exemplary, and some or all of the steps may be carried out by one or more other components of system 10. In various aspects, the disclosed operations can be performed by another component, device, or system. In various aspects, at least some of the operations can be implemented by firmware, programmable logic devices, and/or hardware circuitry. Other implementations are contemplated to be within the scope of the disclosure.

Initially, at step 602, the system 1000 receives a signal from a radio transmitter 200 of a mobile cart 60 supporting a robotic arm 40. The signal received in step 602 includes communication data and localization data of the mobile cart 60 and its components within a 3D space. In aspects, the communication data is in parallel to the communication data. In aspects, the position of the mobile carts 60 in a 3D space is based on the signal communicated by the radio transmitter 200. For In various aspects, the system 1000 may include one or more receivers configured to receive the signal from the radio transmitter 200.

Next, at step 604, the system 1000 determines the location or a spatial pose of the mobile carts 60 based on the localization data component in the received signal. The system 1000 may determine the location or spatial pose of the mobile cart(s) 60 by receiving an indication by the radio receiver 205 of a level of the signal from the radio transmitter 200 or alternatively by extracting the localization data contained in the received signal.

Next, at step 606, the system 1000 determines a destination for the mobile carts 60, that is, a location to move the mobile carts 60, based on the communication data in the received signal. The destination determined in step 606 may be a particular operating room, a storage or docking area, or a specific location within an operating room. In aspects, the determination may be based on a specific surgical procedure, a specific type of patient, a specific type of surgical table, and/or a configuration of the operating room. For example, for a cardiovascular procedure or for a femoral-popliteal procedure, the patient may be in the supine position. For example, for cystoscopy, urology, and/or gynecology procedures, variations of lithotomy position are common. Surgical table accessories such as stirrups, split-leg positioners, and well leg-holders are commonly used to support patient legs during procedures. The surgical table may include additional attachments for these procedures. The surgical table and/or the attachments may include one or more radio transmitters 200 that the system 1000 uses to locate the surgical table and/or attachment when determining the destination for the mobile carts 60.

Step 606 may additionally, or alternatively, include monitoring the battery level of a mobile cart 60 and determining that the location to move the mobile cart 60 (e.g., the destination) be a charging station. The communication data in the received signal may include data corresponding to a power level of a power supply of the mobile cart 60. If the power lever is below a preconfigured threshold, then the destination determined in step 606 may be a charging dock. If the system 1000 determines that a particular mobile cart 60 does not contain a sufficient power supply to perform a surgical procedure to which it is assigned, then the system 1000 may determine that the mobile cart 60 be moved to a charging station to charge the mobile cart's 60 power supply. In this case, method 600 may proceed directly to step 608a, where the mobile cart 60 receives a command to move to a charging station/dock.

Step 606 may additionally, or alternatively, include determining whether the mobile cart 60 requires servicing. The communication data in the received signal may include data corresponding to service data of the mobile cart 60. If the service data in the communication data of the signal indicates that the mobile cart 60 requires servicing or cannot perform the designated procedure without the need for servicing during the procedure, then the destination is determined to be a servicing center. If the system 1000 determines that a particular mobile cart 60 requires servicing or troubleshooting, or otherwise is not performing as expected, then the system 1000 may determine that the mobile cart 60 be moved to a servicing center for technical diagnosis, repairs, or servicing. In this case, method 600 may proceed directly to step 608b, where the mobile cart 60 receives a command to move to a service center.

At step 608a, 608b, 608c, the system 1000 moves the mobile cart(s) 60, causes the mobile cart(s) 60 to move, or presents instructions for a user to move the mobile carts(s) 60 to the location or destination determined in step 606. During movement of the mobile cart(s) 60 to the new location, the system 1000 exchanges data with the mobile cart(s) 60 which includes localization information and tracks the movement of the mobile cart(s) 60 based on the localization data.

As described above, step 606 may include determining that the mobile cart 60 is to be moved to an operating room, or to specific pose within an operating room. In this case, method 600 proceeds to step 608c, where the mobile cart 60 receives a command to move to a specific room or to a specific pose. In step 610, once the mobile cart 60 is moved to the room, the mobile cart 60 is registered to other mobile carts 60 within the surgical setting and to the patient or the surgical table. In step 612, the mobile cart 60 is registered to pre-operative data.

In step 614, one or both of the data storage parameters and the data communication parameters (e.g., data wirelessly communicated between components of the system 10) are adjusted based on the communication data and localization data in the signals received from the mobile carts 60. Adjusting the data storage parameters and/or the data communication parameters reduces bandwidth consumption, thereby reducing power consumption and increasing power and communications efficiency.

In aspects, the robotic arm 40 may include a radio transmitter 200 in operable communication with the radio receiver 205. The system 1000 may receive, from the radio transmitter 200 of the robotic arm 40, a signal including a position of the robotic arm 40 in a 3D space based on the signal communicated by the radio transmitter 200 of the robotic arm 40 and determine the spatial pose of the robotic arm 40 based on the received signal.

In aspects, the robotic arm 40 may include a plurality of individual links, including a plurality of radio transmitters 200 in operable communication with the radio receiver 205. The system 1000 may receive, from the plurality of radio transmitters 200, a plurality of signals including a spatial pose of the plurality of individual links in a 3D space based on the plurality of signals communicated by the plurality of radio transmitters 200 of the individual links. The system 1000 may receive kinematic information from the robotic arm 40 and/or camera positioning information from the robotic arm 40. The system 1000 may receive shape information of the plurality of individual links and cross-reference the spatial pose of the plurality of individual links with the kinematic information and/or camera positioning information. The system 1000 may predict a possible collision with a second robotic arm 40 based on the cross-reference and display an alert, on a display, indicating the possibility of a collision. For example, the system 1000 may automatically set up the spatial pose of the robotic arm 40 based on a combination of the plurality of signals and a desired configuration that is optimal for the procedure.

In various aspects of the disclosure, the operating room staff may wear radio transmitters 200 so that the system 1000 has a spatial awareness of the staff. This assists the system 1000in avoiding collisions between the robotic arm 40 and the staff.

In aspects, the system 1000 may determine whether the mobile cart 60 is in the correct room. This may help with reducing operating room turnaround time and/or locating capital equipment. For example, a particular mobile cart 60 may be in a first operating room, when the system 1000 needs the mobile cart 60 in the second operating room. The system 1000 may include a communication module to allow a node-to-node communication between the mobile carts 60 or the robotic arms 40. For example, the communication module may include 27 Mbps communication between the nodes. The system 1000 would be able to register the nodes by triangulation of the communication modules.

In aspects, the system 1000 may include another radio transmitter 200 located in proximity to a patient (e.g., worn by the patient). In aspects, the system 1000 may determine a spatial pose of the patient based on a signal communicated by the radio transmitter 200 and determine a position of the mobile carts 60 relative to a patient based on the determined spatial pose of the patient.

Following any of steps 608a, 608b, or 612, or at any other point before or after these steps in method 600, the system 1000 may, in step 614, adjust the data storage parameters based on the localization data of the mobile cart 60. In aspects, step 614 includes adjusting what type of information is stored depending on what room the mobile cart 60 is currently in or coordinating the location with the operating room scheduling systems to know what procedure is being performed, and adjusting parameters of the robotic system 1000 accordingly.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The invention may be described by reference to the following numbered paragraphs:-1. A surgical robotic system for radio-based localization and data exchange, the system comprising:
a radio receiver;
a mobile cart including:
   a radio transmitter in operable communication with the radio receiver; and
   a robotic arm;
a processor; and
a memory coupled to the processor, the memory having instructions stored thereon which, when executed by the processor, cause the system to:
   receive, from the radio transmitter, a signal including communication data and localization data of the mobile cart in a 3D space;
   determine a destination for the mobile cart based on the communication data in the received signal; and
   cause the mobile cart to move to the destination determined.
2. The system of paragraph 1, wherein the communication data includes at least one of a specific surgical procedure, a specific type of patient, a specific type of surgical table, or configuration of an operating room and the instructions, when executed, further cause the system to:
   determine the destination for the mobile cart based on at least one of a specific surgical procedure, a specific type of patient, a specific type of surgical table, or configuration of an operating room; and
   cause the mobile cart to move to a new spatial pose.
3. The system of paragraph 1, wherein the signal includes communication data in parallel to the localization data and wherein the radio transmitter is a 5G or 6G radio transmitter capable of transmitting low-band, mid-band, or high-band millimeter waves.
4. The system of paragraph 1, wherein the mobile cart includes a battery power supply.
5. The system of paragraph 4, wherein the communication data includes data corresponding to a power level of the battery power supply and the instructions, when executed, further cause the system to:
   determine whether the power level of the battery power supply is below a preconfigured threshold; and
   cause the mobile cart to move to a charging station when it is determined that the power level of the battery power supply is below the preconfigured threshold.
6. The system of paragraph 1, wherein the communication data includes data corresponding to service data of the mobile cart and the instructions, when executed, further cause the system to:
   determine whether the mobile cart requires servicing based on the communication data; and
   cause the mobile cart to move to a servicing center when it is determined that the mobile cart requires servicing.
7. The system of paragraph 1, wherein the instructions, when executed, further cause the system to register the mobile cart to other mobile carts and to a surgical table based on the communication data and localization data of the mobile cart.
8. The system of paragraph 1, wherein the instructions, when executed, further cause the system to register the mobile cart to pre-operative data based on the communication data and localization data of the mobile cart.
9. The system of paragraph 1, wherein the instructions, when executed, further cause the system to adjust data storage parameters or data communication parameters based on the communication data and localization data of the mobile cart.
10. A method for radio-based localization and data exchange comprising:
   receiving, from a radio transmitter, a signal including communication data and localization data of a mobile cart in a 3D space, wherein the communication data includes data corresponding to a power supply level of the mobile cart and data corresponding to service data of the mobile cart;
   determining a destination for the mobile cart based on the communication data in the received signal;
   causing the mobile cart to move to a charging station when it is determined that a power supply of the mobile cart is below a threshold; and
   causing the mobile cart to move to a servicing center when it is determined that the mobile cart requires servicing.
11. The method of paragraph 10, wherein the communication data includes data corresponding to a specific surgical procedure, a specific type of patient, a specific type of surgical table, or configuration of an operating room, and determining the destination for the mobile cart includes determination the destination based on the data corresponding to a specific surgical procedure, a specific type of patient, a specific type of surgical table, or configuration of an operating room.
12. The method of paragraph 10, further comprising registering the mobile cart to other mobile carts and to a surgical table based on the communication data and localization data of the mobile cart.
13. The method of paragraph 10, further comprising registering the mobile cart to pre-operative data based on the communication data and localization data of the mobile cart.
14. The method of paragraph 10, further comprising adjusting data storage parameters or data communication parameters based on the communication data and the localization data of the mobile cart.
15. A non-transitory computer-readable storage medium storing a program, which when executed by a computer, causes the computer to:
   receive, from a radio transmitter, a signal including communication data and localization data of a mobile cart in a 3D space, wherein the communication data includes data corresponding to a power supply level of the mobile cart;
   determine a destination for the mobile cart based on the communication data in the received signal;
   cause the mobile cart to move to a charging station when it is determined that a power supply of the mobile cart is below a threshold; and
   adjust data storage parameters or data communication parameters based on the communication data and the localization data of the mobile cart.
16. The non-transitory computer-readable storage medium of paragraph 15, wherein the communication data includes at least one of a specific surgical procedure, a specific type of patient, a specific type of surgical table, or configuration of an operating room and the program, when executed by the computer, causes the computer to:
   determine the destination based on at least one of a specific surgical procedure, a specific type of patient, a specific type of surgical table, or configuration of an operating room.
17. The non-transitory computer-readable storage medium of paragraph 15, wherein the program, when executed by the computer, causes the computer to register the mobile cart to other mobile carts and to a surgical table based on the communication data and localization data of the mobile cart.
18. The non-transitory computer-readable storage medium of paragraph 15, wherein the program, when executed by the computer, causes the computer to register the mobile cart to pre-operative data based on the communication data and localization data of the mobile cart.
19. The non-transitory computer-readable storage medium of paragraph 15, wherein the communication data includes data corresponding to service data of the mobile cart and the program, when executed by the computer, causes the computer to determination the destination based on the data corresponding to service data of the mobile cart.
20. The non-transitory computer-readable storage medium of paragraph 15, wherein the program, when executed by the computer, causes the computer to localize and register a position of a surgical instrument to intraoperative imaging based on the communication data and localization data of the mobile cart.

## Claims

1. A surgical robotic system for radio-based localization and data exchange, the system comprising:
a radio receiver;
a mobile cart including:
a radio transmitter in operable communication with the radio receiver; and
a robotic arm;
a processor; and
a memory coupled to the processor, the memory having instructions stored thereon which, when executed by the processor, cause the system to:
receive, from the radio transmitter, a signal including communication data and localization data of the mobile cart in a 3D space;
determine a destination for the mobile cart based on the communication data in the received signal; and
cause the mobile cart to move to the destination determined.

2. The system of claim 1, wherein the communication data includes at least one of a specific surgical procedure, a specific type of patient, a specific type of surgical table, or configuration of an operating room and the instructions, when executed, further cause the system to:
determine the destination for the mobile cart based on at least one of a specific surgical procedure, a specific type of patient, a specific type of surgical table, or configuration of an operating room; and
cause the mobile cart to move to a new spatial pose.

3. The system of claim 1 or claim 2, wherein the signal includes communication data in parallel to the localization data and wherein the radio transmitter is a 5G or 6G radio transmitter capable of transmitting low-band, mid-band, or high-band millimeter waves.

4. The system of any preceding claim, wherein the mobile cart includes a battery power supply; preferably wherein the communication data includes data corresponding to a power level of the battery power supply and the instructions, when executed, further cause the system to:
determine whether the power level of the battery power supply is below a preconfigured threshold; and
cause the mobile cart to move to a charging station when it is determined that the power level of the battery power supply is below the preconfigured threshold.

5. The system of any preceding claim, wherein the communication data includes data corresponding to service data of the mobile cart and the instructions, when executed, further cause the system to:
determine whether the mobile cart requires servicing based on the communication data; and
cause the mobile cart to move to a servicing center when it is determined that the mobile cart requires servicing.

6. The system of any preceding claim, wherein the instructions, when executed, further cause the system to register the mobile cart to other mobile carts and to a surgical table based on the communication data and localization data of the mobile cart and/or wherein the instructions, when executed, further cause the system to register the mobile cart to pre-operative data based on the communication data and localization data of the mobile cart.

7. The system of any preceding claim, wherein the instructions, when executed, further cause the system to adjust data storage parameters or data communication parameters based on the communication data and localization data of the mobile cart.

8. A method for radio-based localization and data exchange comprising:
receiving, from a radio transmitter, a signal including communication data and localization data of a mobile cart in a 3D space, wherein the communication data includes data corresponding to a power supply level of the mobile cart and data corresponding to service data of the mobile cart;
determining a destination for the mobile cart based on the communication data in the received signal;
causing the mobile cart to move to a charging station when it is determined that a power supply of the mobile cart is below a threshold; and
causing the mobile cart to move to a servicing center when it is determined that the mobile cart requires servicing.

9. The method of claim 8, wherein the communication data includes data corresponding to a specific surgical procedure, a specific type of patient, a specific type of surgical table, or configuration of an operating room, and determining the destination for the mobile cart includes determination the destination based on the data corresponding to a specific surgical procedure, a specific type of patient, a specific type of surgical table, or configuration of an operating room; preferably further comprising registering the mobile cart to other mobile carts and to a surgical table based on the communication data and localization data of the mobile cart.

10. The method of claim 9, further comprising registering the mobile cart to pre-operative data based on the communication data and localization data of the mobile cart.

11. The method of claim 9 or claim 10, further comprising adjusting data storage parameters or data communication parameters based on the communication data and the localization data of the mobile cart.

12. A non-transitory computer-readable storage medium storing a program, which when executed by a computer, causes the computer to:
receive, from a radio transmitter, a signal including communication data and localization data of a mobile cart in a 3D space, wherein the communication data includes data corresponding to a power supply level of the mobile cart;
determine a destination for the mobile cart based on the communication data in the received signal;
cause the mobile cart to move to a charging station when it is determined that a power supply of the mobile cart is below a threshold; and
adjust data storage parameters or data communication parameters based on the communication data and the localization data of the mobile cart; preferably wherein the communication data includes at least one of a specific surgical procedure, a specific type of patient, a specific type of surgical table, or configuration of an operating room and the program, when executed by the computer, causes the computer to:
determine the destination based on at least one of a specific surgical procedure, a specific type of patient, a specific type of surgical table, or configuration of an operating room.

13. The non-transitory computer-readable storage medium of claim 12 wherein the program, when executed by the computer, causes the computer to register the mobile cart to other mobile carts and to a surgical table based on the communication data and localization data of the mobile cart; preferably wherein the program, when executed by the computer, causes the computer to register the mobile cart to pre-operative data based on the communication data and localization data of the mobile cart.

14. The non-transitory computer-readable storage medium of claim 13 or claim 14,
wherein the communication data includes data corresponding to service data of the mobile cart and the program, when executed by the computer, causes the computer to determination the destination based on the data corresponding to service data of the mobile cart.

15. The non-transitory computer-readable storage medium of any of claim 12 to 14,
wherein the program, when executed by the computer, causes the computer to localize and register a position of a surgical instrument to intraoperative imaging based on the communication data and localization data of the mobile cart.
